# EUROPEAN PATENT APPLICATION

(11) **EP 4 279 030 A1**
(43) Date of publication of application: **22.11.2023**
(21) Application number: 21919081.6
(22) Date of filing: 14.12.2021
(51) Int. Cl.: A61F 2/24

(54) **BALLOON-EXPANDABLE INTERVENTIONAL VALVE STENT PROVIDED WITH FOLDABLE WINGSPAN SKIRT**

(30) Priority: 14.01.2021 CN 202110051095
(71) Applicant: Nanjing Saint Medical Technology Co., Ltd., Nanjing, Jiangsu 210061 (CN)
(72) Inventor: MA, Chenming, Nanjing, Jiangsu 210061 (CN); GU, Jiajia, Nanjing, Jiangsu 210061 (CN)
(74) Representative: Gille Hrabal Partnerschaftsgesellschaft mbB Patentanwälte
(86) International application number: PCT/CN2021/137953
(87) International publication number: WO 2022/151892

(57) **Abstract**

A balloon-expandable interventional valve stent provided with a foldable wingspan skirt (2), comprising a stent body (1), a valve leaflet, and a wingspan skirt (2). The wingspan skirt (2) is disposed on the outer side of an outflow tract (4); a pull suture (5) is connected to the wingspan skirt (2); when a balloon (7) is expanded and opened, the pull suture (5) connected to the skirt on the side of an inflow tract (3) is gradually tightened in a circumferential direction under the action of expanding force of the balloon (7), so that the wingspan skirt (2) is folded in the axis direction of the stent body (1), and a portion protruding outwards in the radial direction of the stent body (1) is formed, thus covering or plugging a paravalvular leak. A wingspan skirt (2) structure that can be utilized is added without increasing the diameter of a delivery system, thus greatly improving a plugging effect of the paravalvular leak.

## Description

The present application claims priority to Patent Application No. 202110051095.X filed with China National Intellectual Property Administration on Jan. 14, 2021 and entitled "BALLOON-EXPANDABLE INTERVENTIONAL VALVE STENT PROVIDED WITH FOLDABLE WINGSPAN SKIRT", which is incorporated herein by reference in its entirety.

### TECHNICAL FIELD

The present disclosure belongs to the field of medical devices, and particularly relates to a balloon-expandable interventional valve stent with a foldable wing-spreading skirt, which can be applied to aortic valve, pulmonary valve, mitral valve and tricuspid valve positions, damaged biological valve, valve in valve, and other scenarios.

### BACKGROUND

Paravalvular leak (PVL), i.e., a leak formed between an artificial valve and surrounding tissues, is a common and serious complication in all heart valve surgeries, particularly the incidence of which is extremely high in transcatheter valve therapies that are currently becoming more prevalent. Several studies have shown that postsurgical paravalvular leaks are directly related to the degree of recovery, reoperation rate, hospital readmission rate, and early-, medium-, and long-term mortality of a patient. A meta-analysis of several international studies has shown that only 6%-59% of patients after the transcatheter aortic valve interventional surgery did not develop paravalvular leaks. In addition to unavoidable reasons such as the inability to remove the patient's original degenerated, decayed, or damaged valves, and the patient's own anatomical structures such as a non-circular valve annulus plane and other factors, the reasons for this are also closely related to the design characteristics of the current transcatheter valve interventional surgical equipment. With the popularization of transcatheter technology all over the world, more patients, especially younger patients, will choose transcatheter valve surgery to receive treatment, and the emergence of novel transcatheter valve interventional surgical equipment is imperative.

Currently, the existing balloon-expandable interventional valve is usually provided with a skirt on the outer side of an inflow channel, such as the artificial valve structure disclosed in Patent Document CN108430394A, and such structure is designed based on years of clinical experience, and it is considered that the paravalvular leak can be improved by increasing the contact area with the heart valve annulus plane. However, the existing valve structure still has a problem in that the effectiveness of paravalvular leak prevention is limited because the size of the valve delivery system cannot be made too large, resulting in the skirt not being able to be infinitely thickened. Therefore, there is a need for an interventional valve that can prevent the paravalvular leak without increasing the size of the valve delivery system.

### SUMMARY

Therefore, an object of the present disclosure is to provide a balloon-expandable interventional valve stent with a foldable wing-spreading skirt. According to a special design, a foldable wing-spreading skirt is arranged on an outflow channel of a stent, and the wing-spreading skirt can extend radially outwards from the stent to cover and occlude a paravalvular leak, so that adverse effects caused by the paravalvular leak are prevented.

The present disclosure is implemented by the following technical schemes:
The present disclosure provides a balloon-expandable interventional valve stent with a foldable wing-spreading skirt, comprising a stent main body, a valve leaflet, and a wing-spreading skirt, wherein the stent main body comprises an inflow channel positioned at a lower portion and an outflow channel positioned at an upper portion, the wing-spreading skirt is arranged on an outer side of the outflow channel, the side of the wing-spreading skirt close to a middle portion of the stent main body is fixedly connected to the stent main body, other portions of the wing-spreading skirt are not fixedly connected to the stent main body, and the surface of the wing-spreading skirt is attached to the surface of the stent main body; an inflow channel side of the stent main body is also provided with a skirt; a pulling suture is connected to the wing-spreading skirt, one side of the pulling suture is connected to the wing-spreading skirt, the other side is connected to the skirt on the inflow channel side, and the pulling suture is connected between the wing-spreading skirt and the skirt on the inflow channel side in a reciprocating manner for multiple times; when a balloon is expanded and spread, two ends of the stent main body are firstly spread, and the middle portion of the stent main body is subsequently spread; when the stent main body on the inflow channel side is spread, the skirt on the inflow channel side is unfolded, and the pulling suture connected to the skirt on the inflow channel side is gradually tightened in a circumferential direction under the action of an expansion force of the balloon, such that the pulling suture in the wing-spreading skirt on an outflow channel side is tensioned, and the tensioned pulling suture causes the wing-spreading skirt to be folded in an axial direction of the stent main body to form a portion protruding outwards in a radial direction of the stent main body, thereby covering or occluding a paravalvular leak.

According to the valve stent described herein, the pulling suture connected in the wing-spreading skirt is integrally in a plurality of continuous shapes like a Chinese character "ji (JL)", and the pulling suture is alternately arranged on an inner side and an outer side of the wing-spreading skirt for multiple times, such that the pulling suture on the inner side surface and the outer side surface of the wing-spreading skirt is in a dotted line shape; when the pulling suture is tensioned, the wing-spreading skirt folded in the axial direction of the stent main body is in a multi-layer fold shape, and the wing-spreading skirt in the folded state is in a wing-spreading shape outwards in the radial direction of the stent main body under the combined action of the expansion force of the balloon and the tension force of the pulling suture.

According to the valve stent described herein, the number of the pulling suture is 1, 2, 3, 4, 6, or 12, preferably 1, and the pulling suture continuously passes through a plurality of connecting points in the wing-spreading skirt and on the skirt of the inflow channel side.

According to the valve stent described herein, when there are 2 or more pulling sutures, the 2 or more pulling sutures described above have equal length to each other, are connected end to end in succession at the connecting points of the skirt on the inflow channel side, and jointly cover the whole circumference of the wing-spreading skirt.

According to the valve stent described herein, the wing-spreading skirt and the pulling suture are both made of a flexible non-metallic material.

According to the valve stent described herein, the pulling suture is made of a polymer material, preferably polyglycolic acid, polylactic acid, or poly(p-dioxanone).

The beneficial effects of the present disclosure are as follows:
The present disclosure has an advantage that the structure of the outflow channel is fully utilized, such that the wing-spreading skirt that can be used is increased on the premise of not increasing the diameter of a delivery system. For the paravalvular leak, such as a non-elliptical heart valve annulus, the wing-spreading skirt can provide a superior occlusion effect relative to the current prior art. Moreover, because of the use of flexible non-metallic materials, the additional structure does not increase damage to the heart.

### BRIEF DESCRIPTION OF THE DRAWINGS

FIG. 1 is a schematic view of a balloon-expandable interventional valve stent in a compressed state according to an embodiment of the present disclosure;
FIG. 2 is a schematic view of the balloon-expandable interventional valve stent during an expansion process according to an embodiment of the present disclosure;
FIG. 3 is a schematic view of the balloon-expandable interventional valve stent in an expanded state according to an embodiment of the present disclosure;
FIG. 4 is a schematic view of pulling sutures of the balloon-expandable interventional valve stent according to an embodiment of the present disclosure;
FIG. 5 is a front view of an effect view of the balloon-expandable interventional valve stent of the present disclosure; and
FIG. 6 is a top view of the effect view of the balloon-expandable interventional valve stent of the present disclosure.

### DETAILED DESCRIPTION

The present disclosure will be further illustrated with reference to the following specific examples. It should be understood that these examples are merely intended to illustrate the present disclosure rather than limit the protection scope of the present disclosure. In addition, it should be understood that various changes or modifications may be made by those skilled in the art after reading the teachings of the present disclosure, and these equivalents also fall within the protection scope of the present disclosure.

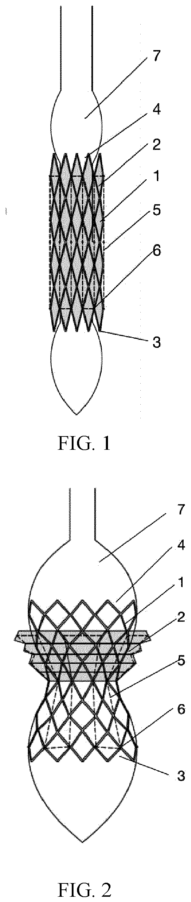

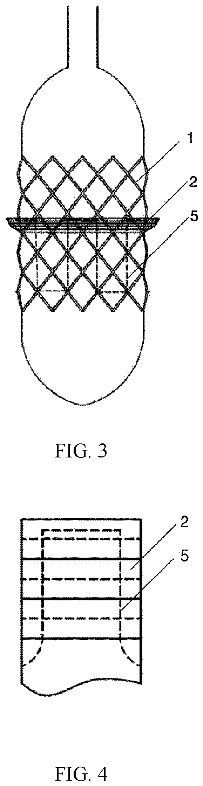

As shown in FIGs. 1-3, a balloon-expandable interventional valve stent comprises a stent main body 1, a valve leaflet (not shown), and a wing-spreading skirt 2, wherein the stent main body 1 comprises an inflow channel 3 positioned at a lower portion and an outflow channel 4 positioned at an upper portion, the wing-spreading skirt 2 is arranged on an outer side of the outflow channel 4, the side of the wing-spreading skirt 2 close to a middle portion of the stent main body 1 is fixedly connected to the stent main body 1, other portions are not fixed, and the surface of the wing-spreading skirt 2 is attached to the surface of the stent main body 1. A pulling suture 5 is connected to the wing-spreading skirt 2, the pulling suture 5 is integrally in a plurality of continuous shapes like a Chinese character "ji (JL)" (as shown in FIG. 4), and the pulling suture 5 is alternately arranged on an inner side and an outer side of the wing-spreading skirt for multiple times, such that the suture on the inner side surface and the outer side surface of the wing-spreading skirt is in a dotted line shape.

An inflow channel side of the stent main body 1 is also provided with a skirt (not shown in the figures), and the pulling suture 5 is connected to the outer skirt on the inflow channel side. There can be several pulling sutures 5, for example, 1, 2, 3, 4, 6, or 12 pulling sutures, preferably 1 pulling suture, and the pulling suture continuously passes through a plurality of connecting points 6 in the wing-spreading skirt 2 and on the outer skirt of the inflow channel 3 side; when there are 2 or more pulling sutures 5, the 2 or more pulling sutures 5 described above have equal length to each other, are connected end to end in succession at the connecting points 6, and jointly cover the whole circumference of the wing-spreading skirt 2.

When a balloon 7 is expanded and spread, two ends of the stent main body 1 are firstly spread, and the middle portion of the stent main body 1 is subsequently spread; when the stent main body 1 on the inflow channel side is spread, the skirt on the inflow channel side is unfolded, the pulling suture 5 connected to the skirt on the inflow channel side is gradually tightened in a circumferential direction under the action of an expansion force of the balloon, such that the pulling suture 5 in the wing-spreading skirt 2 on the outflow channel side is tensioned, causing the wing-spreading skirt 2 on the outflow channel side to be folded in an axial direction of the stent main body 1, the wing-spreading skirt 2 in the folded state presents a wing-spreading shape outwards in a radial direction of the stent main body 1 under the combined action of the expansion force of the balloon and the tension force of the pulling suture, and the skirt portion extending outwards in the radial direction can cover and occlude a paravalvular leak and integrally form the foldable wing-spreading skirt. The wing-spreading skirt 2 is made of a flexible non-metallic material, and the pulling suture 5 can be made of a polymer material such as polyglycolic acid, polylactic acid, poly(p-dioxanone), or the like.

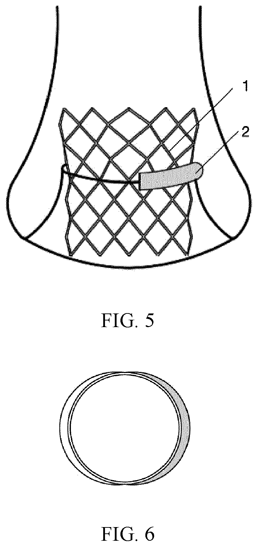

As can be seen from FIGs. 5 and 6, the technical scheme of the present disclosure makes full use of the structure of the outflow channel, such that the wing-spreading skirt that can be used is increased on the premise of not increasing the diameter of a delivery system. The wing-spreading skirt structure of the present disclosure can greatly improve the occlusion effect on the paravalvular leak. Moreover, because of the use of flexible non-metallic materials, the additional structure does not increase damage to the heart.

The embodiments of the present disclosure have been described above. However, the present disclosure is not limited to the embodiments described above. Any modification, equivalent, improvement, and the like made without departing from the spirit and principle of the present disclosure shall fall within the protection scope of the present disclosure.

## Claims

1. A balloon-expandable interventional valve stent with a foldable wing-spreading skirt, comprising a stent main body, a valve leaflet, and a wing-spreading skirt, wherein the stent main body comprises an inflow channel positioned at a lower portion and an outflow channel positioned at an upper portion, the wing-spreading skirt is arranged on an outer side of the outflow channel, the side of the wing-spreading skirt close to a middle portion of the stent main body is fixedly connected to the stent main body, other portions of the wing-spreading skirt are not fixedly connected to the stent main body, and the surface of the wing-spreading skirt is attached to the surface of the stent main body;
an inflow channel side of the stent main body is also provided with a skirt; a pulling suture is connected to the wing-spreading skirt, one side of the pulling suture is connected to the wing-spreading skirt, the other side is connected to the skirt on the inflow channel side, and the pulling suture is connected between the wing-spreading skirt and the skirt on the inflow channel side in a reciprocating manner for multiple times;
when a balloon is expanded and spread, two ends of the stent main body are firstly spread, and the middle portion of the stent main body is subsequently spread; when the stent main body on the inflow channel side is spread, the skirt on the inflow channel side is unfolded, and the pulling suture connected to the skirt on the inflow channel side is gradually tightened in a circumferential direction under the action of an expansion force of the balloon, such that the pulling suture in the wing-spreading skirt on an outflow channel side is tensioned, and the tensioned pulling suture causes the wing-spreading skirt to be folded in an axial direction of the stent main body to form a portion protruding outwards in a radial direction of the stent main body, thereby covering or occluding a paravalvular leak.

2. The balloon-expandable interventional valve stent with the foldable wing-spreading skirt according to claim 1, wherein the pulling suture connected in the wing-spreading skirt is integrally in a plurality of continuous shapes like a Chinese character "ji (JL)", and the pulling suture is alternately arranged on an inner side and an outer side of the wing-spreading skirt for multiple times, such that the pulling suture on the inner side surface and the outer side surface of the wing-spreading skirt is in a dotted line shape; when the pulling suture is tensioned, the wing-spreading skirt folded in the axial direction of the stent main body is in a multi-layer fold shape, and the wing-spreading skirt in the folded state is in a wing-spreading shape outwards in the radial direction of the stent main body under the combined action of the expansion force of the balloon and the tension force of the pulling suture.

3. The balloon-expandable interventional valve stent with the foldable wing-spreading skirt according to claim 2, wherein the number of the pulling suture is 1, 2, 3, 4, 6, or 12, preferably 1, and the pulling suture continuously passes through a plurality of connecting points in the wing-spreading skirt and on the skirt of the inflow channel side.

4. The balloon-expandable interventional valve stent with the foldable wing-spreading skirt according to claim 3, wherein when there are 2 or more pulling sutures, the 2 or more pulling sutures described above have equal length to each other, are connected end to end in succession at the connecting points of the skirt on the inflow channel side, and jointly cover the whole circumference of the wing-spreading skirt.

5. The balloon-expandable interventional valve stent with the foldable wing-spreading skirt according to claim 4, wherein the wing-spreading skirt and the pulling suture are both made of a flexible non-metallic material.

6. The balloon-expandable interventional valve stent with the foldable wing-spreading skirt according to claim 5, wherein the pulling suture is made of a polymer material, preferably polyglycolic acid, polylactic acid, or poly(p-dioxanone).
